# EUROPÄISCHE PATENTANMELDUNG

(11) **EP 4 202 055 A1**
(43) Veröffentlichungstag der Anmeldung: **28.06.2023**
(21) Anmeldenummer: 21216680.5
(22) Anmeldetag: 21.12.2021
(51) Int. Cl.: C12Q 1/22, C12Q 1/48, C12Q 3/00

(54) **ENZYMINDIKATOREN FÜR DIE PROZESSKONTROLLE VON STERILISATIONSPROZESSEN**

(71) Anmelder: Bayer Aktiengesellschaft, 51373 Leverkusen (DE)
(72) Erfinder: Die Erfindernennung liegt noch nicht vor
(74) Vertreter: BIP Patents

(57) **Zusammenfassung**

Die Erfindung betrifft die Verwendung von Enzymindikatoren für die Prozesskontrolle eines Sterilisationsverfahrens oder für die Entwicklung oder Validierung eines Sterilisationsverfahrens, sowie Verfahren für die Entwicklung, Validierung und Prozesskontrolle von Sterilisationsverfahren. Die Sterilisationsverfahren dienen dabei der Sterilisation der Außenfläche von Primärbehältern, welche sich in einem funktionalen Packmittel befinden. Weiterhin betrifft die Erfindung funktionale Packmittel, welche einen Primärbehälter und einen Enzymindikator umfassen.

## Beschreibung

Die Erfindung betrifft die Verwendung von Enzymindikatoren zur Prozessentwicklung und Prozesskontrolle in der Oberflächensterilisation von Primärbehältern, welche sich in funktionalen Packmitteln befinden. Weiterhin betrifft die Erfindung funktionale Packmittel, welche einen Primärbehälter und einen Enzymindikator umfassen und ein Verfahren zur Prozesskontrolle der Oberflächensterilisation unter der Verwendung von Enzymindikatoren.

### Problem

Einige Kombinations- und Medizinprodukte müssen am Ende des Herstellungsprozesses sterilisiert werden, um eine sichere Anwendung am Patienten zu gewährleisten. Behälter, welche medizinische Produkte enthalten (z.B. vorgefüllte Spritzen, Kartuschen, Phiolen) stellen dabei eine Besonderheit dar, da sie auf ihrer Außenfläche bis zum Zeitpunkt der Anwendung am Patienten steril sein müssen, um eine sichere Anwendung zu gewährleisten. Dazu werden diese Produkte in einer weiteren Verpackung eingefasst, welche für Pathogene undurchlässig ist, jedoch von verdampften oder gasförmigen Sterilisationsagenzien durchdrungen werden kann. Somit kann die Außenfläche des Behälters sterilisiert werden, wenn der Behälter sich in der Verpackung befindet. Darüber hinaus verhindert die Verpackung eine Oberflächenkontamination nach der Sterilisation und die Sterilität des verpackten Kombinations- oder Medizinprodukts bleibt bis zur Anwendung erhalten.

Zur Validierung und Kontrolle von Sterilisationsprozessen zur Herstellung von Arzneimitteln, Kombinations- und Medizinprodukten werden biologischer Indikatoren (BI) genutzt. (European Pharmacopoeia 10.7, 5.1.1. Methods of preparation of sterile products, 07/2017:50101, 2017.) Dabei kommen Sporen thermostabiler Bakterien zum Einsatz, von denen üblicherweise 10⁶ bis 10⁷ auf einem festen Träger immobilisiert sind. Der Sporenträger ist von einer gasdurchlässigen Hülle umgeben, sodass das gasförmige oder verdampfte Sterilisationsagens die Sporen erreichen kann, ohne dass das Sterilisationsgut den Sporen direkt ausgesetzt ist. Nach der Sterilisation wird die Hülle entfernt und der Sporenträger wird in eine Nährlösung gegeben, welche bei geeigneten Bedingungen inkubiert wird. Sofern sich auf dem Träger noch überlebensfähige Sporen befinden, vermehren sich diese und trüben die Lösung ein. Geschieht dies im Rahmen einer routinemäßigen Sterilisation, gilt diese in der Regel als nicht bestanden, da die behördlich vorgeschriebene Sterilisationseffizienz von 6 Logstufen nicht nachgewiesen werden konnte. (European Pharmacopoeia 10.7, 5.1.2. Biological indicators and related microbial preparations used in the manufacture of sterile products, 07/2017:50102, 2017).

Biologische Indikatoren (BI) unterliegen zahlreichen Nachteilen. Neben der langen Inkubationsdauer, welche die Entwicklung von Sterilisationszyklen und die Freigabe sterilisierter Marktware verzögert, erschwert die Heterogenität der Resistenz und Population zwischen einzelnen Indikatoren und verschiedenen Indikatorchargen die Prozessentwicklung, die bewusst unter Bedingungen erfolgt, welche ein teilweises Auswachsen mehrerer im Sterilisationsgut platzierter Bioindikatoren erfordert (Fraktion-Negativ-Verfahren). (DIN Deutsches Institut für Normung e. V., Sterilisation von Produkten für die Gesundheitsfürsorge -Biologische Indikatoren -Teil 1: Allgemeine Anforderungen (ISO 11138-1:2017);Deutsche Fassung EN ISO 11138-1:2017, Berlin: Beuth Verlag GmbH, 2017). Die geringe Robustheit des Herstellprozesses spiegelt sich im breiten Akzeptanzkriterium bei der Populationsbestimmung wider (50-300% Wiederfindung). Das rein binäre Ergebnis der Bebrütung (Wachstum/kein Wachstum) gibt zudem nur geringen Aufschluss über die Sterilisationseffizienz. Weiterhin kann es durch den Herstellprozess oder mechanische Belastung der Sporenträger zur Verklumpung von Sporen kommen, welche die Abtötung von Sporen im Innern erschweren und somit zu falsch positiven Testergebnissen führen. Dies kann zum Verwerfen ganzer Sterilisationschargen führen und damit zu einer eingeschränkten Patientenversorgung.

### Aufgabe

Es besteht daher ein Bedarf nach einer zuverlässigen Methode die, die Sterilität von Außenflächen von Kombinationsprodukten nachweisen kann und somit für die Entwicklung von Sterilisationsprozessen und für Freigabeverfahren verwendet werden kann. Die Methode soll nicht die Nachteile der Verwendung von biologischen Indikatoren aufweisen und soll den Nachweis einer Sterilität von bis zu 12-Log Reduktion ermöglichen, um den behördlichen Anforderungen gerecht zu werden.

### Stand der Technik

Als Alternative zu BIs wurden in den vergangenen Jahren enzymatische Indikatoren (EIs) entwickelt. Die Immobilisation stabiler Enzyme auf Trägern, welche unmittelbar im Anschluss an die Sterilisation mittels eines quantitativen, luminometrischen Tests ausgelesen werden, können Prozessentwicklungen oder Freigabeverfahren signifikant beschleunigen. Das Herstellverfahren garantiert zudem eine weitaus geringere Heterogenität zwischen einzelnen Indikatoren.

WO2005/093085 (Health Protection Agency) beschreibt Kinasen insbesondere thermostabile Kinasen als verbesserte Indikatoren im Vergleich zu herkömmlichen biologischen Indikatoren für Sterilisationsprozesse. Sie zeigen eine verbesserte Sensitivität gegenüber herkömmlichen biologischen Indikatoren, da sie eine Verminderung der Aktivität über 6 log hinaus bis zu 9 logs anzeigen können. Die beschriebenen Enzymindikatoren wurden für folgende Sterilisationsprozesse/Dekontaminationsprozesse beschrieben: Waschen von Textilien mit biologischen Detergenzien, Dekontamination von harten Oberflächen, Dekontamination mittels Ozons und Dekontamination mittels flüssigen chemischen Agenzien, Dekontamination von Flüssigkeiten. In all diesen Dekontaminationsprozessen sind die zu dekontaminierenden Objekte direkt für das Sterilisationsagenz zugänglich und nicht durch ein funktionales Packmittel abgeschirmt. Die Verwendung der Indikatoren für die Oberflächensterilisation von Primärbehältern, welche eine Flüssigkeit enthalten und sich in einem funktionalen Packmittel befinden, ist nicht beschrieben.

WO2009/104013 (Health Protection Agency) beschreibt biologische Indikatoren, bestehend aus Enzymen, welche auf biologischem Material immobilisiert sind und deren Verwendung für die Validierung eines Dekontaminationsprozesses, welcher geeignet ist, die Aktivität einer biologischen Verunreinigung in einer Probe zu verringern. Solche Dekontaminationsprozesse können z.B. Waschschritte für Textilien sein, die in Krankenhäusern verwendet werden, Desinfektion von Gegenständen mit flüssigen Desinfektionsmitteln, oder Sterilisation von Flüssigkeiten sein. Dabei wird eine Verminderung der Aktivität oder Menge der Verunreinigung mindestens 3 - 9 log beschrieben. Weiterhin wird die Sterilisation von Außenflächen, die vollständig verpackt sind beschrieben. Da die Verpackung eine Penetrationsbarriere für das Sterilisationsagens darstellt, werden die Enzyme auf porösen Materialien immobilisiert oder in Gelen oder Harzen verkapselt, welche ein Surrogat für die Diffusionsbarriere darstellen, und somit die Verpackung imitieren. Die Anmeldung offenbart jedoch nicht die Platzierung der EI in die Verpackung, um die tatsächliche Zugänglichkeit eines verpackten Medizin- oder Kombinationsprodukts nachzuahmen.

McLeod et al. PDA J Pharm Sci Technol.; 2017;71(5):393-404 (10.5731/pdajpst.2016.007435 [14]) beschreibt die Verwendung einer thermostabilen Adenylat-Kinase als Enzymindikator im Vergleich zu kommerziellen 6-log biologischen Indikatoren in der Dampfphasen-Biodekontamination mit Wasserstoffperoxid von Reinheits-Räumen wie z.B. Isolatoren. Dabei konnte eine vergleichbare 6-log Verminderung der Dekontamination mit beiden Indikatoren beobachtet werden.

Schachtschneider et al. J Pharm Sci Technol. 2021 (10.573 1/pdajpst.2020.012294) untersucht die Vergleichbarkeit von einer thermostabilen Adenylat-Kinase als Enzymindikator mit einem kommerziellen biologischen Indikator *(Geobacillus stearothermophilus* auf unbeschichteten Stahlscheiben) in der Sterilisation von Isolatoren mit Wasserstoffperoxid Biodekontaminationssystemen. Dabei konnte eine vergleichbare 6-log Verminderung der Dekontamination mit beiden Indikatoren beobachtet werden.

Es besteht daher ein Bedarf an technischen Lösungen für die Sterilisationskontrolle von der Sterilisation von Außenflächen, welche vollständig verpackt sind.

### Lösung

Die Aufgabe wird gelöst durch Demonstration der Eignung von Enzymindikatoren zur Entwicklung und Kontrolle eines Sterilisationsverfahrens, welches eine 12-log Reduktion ermöglicht, und durch die geeignete Handhabung und Platzierung des Enzymindikators innerhalb des funktionalen Packmittels. Der EI ist somit an einer für das Sterilisationsgut repräsentativen Stelle positioniert und bildet die Herausforderung der Penetration des Sterilisationsagens durch die funktionale Verpackung effektiv ab.

Die kürzeren Auswertungszeiten der EIs könnten die Chargenfreigabe maßgeblich beschleunigen. Das weit weniger aufwändige und platzsparende Verfahren vereinfacht zudem die Qualitätskontrolle und spart Kosten ein. Das robustere Messverfahren reduziert das Risiko falsch-positiver Prozesskontrollen und erlaubt eine zielgerichtetere Prozessentwicklung und - optimierung.

Überraschenderweise konnte gezeigt werden, dass
- EI mechanisch stabil sind und damit robust genug um in ein funktionales Packmittel eingebracht zu werden
- EI über einen längeren Zeitraum bei Raumtemperatur gelagert werden können
- EI nach der Sterilisation zeitverzögert ausgelesen werden können
- EI den Sterilisationserfolg nach der Sterilisation in einem funktionalen Packmittel mit vergleichbarer Qualität anzeigen können wie biologische Indikatoren.

### Definitionen

Der Begriff **"Kontaminant", oder "Kontamination"** oder "Verunreinigung" umfasst sowohl infektiöse als auch nicht infektiöse Agenzien, die aus einer biologischen Quelle stammen. Beispiele für Kontaminanten umfassen Bakterien, Viren, Pilze, Prionen, Toxine, Allergene, Sporen. Die Verunreinigungen können sich auf festen Oberflächen, Hohlräumen, in Flüssigkeiten oder in der Luft befinden. Das zu sterilisierende Objekt bzw. die zu sterilisierende Oberfläche wird als "Sterilisationsgut" bezeichnet.

Der Begriff **"Behandlung", oder "Behandlungsverfahren"** umfasst jedes Verfahren, das darauf ausgelegt ist, die Menge oder Aktivität einer Verunreinigung in einer Probe zu verringern. "Dekontamination" oder "Dekontaminationsverfahren" ist hier spezifisch als Verfahren definiert, das eine Abreicherung lebensfähiger, resistenter Sporen um mindestens drei Logstufen ermöglicht.

Die Verfahrensentwicklung setzt die Bestimmung des prozessspezifischen "D-Wertes" eines adäquat gewählten Bioindikators voraus. Der D-Wert kann die Zeit, die Injektionsmenge oder die Anzahl an Injektionspulsen beschreiben, die benötigt wird, um die auf dem Indikator befindliche Population um 90% zu reduzieren. Die Bestimmung dieses D-Werts kann beispielsweise mittels Charakterisierung einer Überlebenskurve oder durch das Fraktion-Negativ-Verfahren erfolgen. (DIN Deutsches Institut für Normung e. V., Sterilisation von Produkten für die Gesundheitsfürsorge -Biologische Indikatoren -Teil 1: Allgemeine Anforderungen (ISO 11138-1:2017);Deutsche Fassung EN ISO 11138-1:2017, Berlin: Beuth Verlag GmbH, 2017) Auf Basis des D-Wertes kann die Prozessauslegung gemäß der angestrebten Log-Reduktion erfolgen. **"n Log Reduktion" oder "n log" oder "n Log"** bezeichnet die Reduktion der Anzahl von Kontaminanten um 90 Prozent bezogen auf den Ausgangswert. Eine n-fache Logreduktion entspricht somit einer Reduktion der Kontaminanten auf das 10⁻ⁿ-fache des Ausgangswerts.

Der Begriff **"Sterilisationsverfahren" oder "Sterilisation"** beschreibt Verfahren, welche der vollständigen Inaktivierung lebensfähiger Kontaminanten dienen und ein Sterilisationssicherheitsniveau von mindestens 10⁻⁶ (6 Logstufen) bieten. Hierbei kommen hohe Temperaturen, hohe Drücke, energiereiche Strahlung (z.B. UV- oder Gammastrahlung), flüssige, gasförmige oder verdampfte chemische Agenzien (Wasserstoffperoxid, Ethylenoxid, Stickstoffdioxid, Formaldehyd) zum Einsatz. Sterilisationsagenzien können z.B. verdampfte Agenzien wie z.B. Wasserstoffperoxid oder gasförmige Agenzien wie z.B. Ethylenoxid, Formaldehyd oder Stickstoffdioxid sein. Gasförmige oder verdampfte chemische Sterilsationsagenzien ermöglichen eine Niedertemperatursterilisation, und haben einen inaktivierenden Effekt auf das Indikatorenzym. Das Einbringen der Gase in eine geeignete Sterilisationskammer kann durch mehrfache, also pulsweise, Injektion und, falls notwendig, unter Veränderung des Kammerdrucks erfolgen. Eine weitere Alternative ist eine einmalige Injektion und genaue Dosierung über die Einwirkdauer.

Unter den Sterilisationsverfahren stellen "Overkillverfahren" eine Sonderform dar, da sie nicht den tatsächlichen Grad der Kontamination des Sterilisationsguts berücksichtigen, sondern über das Maß hinausgehen, das zur Erfüllung der festgelegten Anforderungen an die Sterilität erforderlich ist. Ein Overkillverfahren erreicht somit mindestens eine Reduktion der Kontaminanten um 12 Logstufen.

Sterilisationsverfahren sind z.B. beschrieben in US10905786 (Regeneron), EP0302420A2 (Surgicos) und WO2014187779 (Bayer). So beschreibt z.B. US10905786 eine Sterilisationskammer, welche für die Außenflächen-Sterilisation von medizinischen Produkten und Geräten. EP0302420A2 beschreibt ein Niedertemperaturverfahren unter Nutzung verdampften Wasserstoffperoxids. WO2014187779A1 kombiniert die Nutzung eines solchen Verfahrens mit Wasserstoffperoxid mit einem funktionalen Packmittel.

Der Begriff **"funktionales Packmittel"** umfasst Erzeugnisse, die zur Verpackung von Produkten verwendet werden. Ein funktionales Packmittel kann eine flexible Verpackung (Pouch, Tüte, Folie) oder eine steife Verpackung (z.B. Blister) oder eine Kombination dieser beiden sein. Ein funktionale Packmittel ermöglicht eine Sterilisation und wahrt die Sterilität mindestens über die Lebensdauer des Produktes. Das funktionale Packmittel wird gemäß ISO 11607-1 und -2 entwickelt. Das funktionale Packmittel umschließt den Primärbehälter vollständig und kann aus ein oder zwei oder mehreren Materialien bestehen, die gegeneinander fest verbunden bzw. verschlossen werden, z.B. gesiegelt sind. Mindestens eines der Materialien ist durchlässig für Gase, insbesondere Sterilisationsgase (wie Wasserstoffperoxid oder Ethylenoxid). Andererseits ist dieses Material keimundurchlässig. Ein solches gasdurchlässiges, aber keimundurchlässiges Material kann z.B.: Polyethylen-Fließ (PE-Fließ z.B. Handelsmarke Tyvek) oder Papier bzw. Abwandlungen von zuvor genannten Materialien sein. Das funktionale Packmittel kann eine Form haben, die sicherstellt, dass der Primärbehälter in seiner Position verbleibt. Das funktionale Packmittel kann auch verhindern, dass bei Druckschwankungen, z.B. während des Transportes mit dem Flugzeug oder bei der Sterilisation, flexible Komponenten, wie z.B. der Stopfen der Spritze, sich nicht verschieben.

Der Begriff **"Primärbehälter"** umfasst jede Art von Behälter, der ein pharmazeutisches Produkt aufnehmen kann und in dem das Produkt über einen längeren Zeitraum gelagert werden kann, ohne dass Kontaminanten die Flüssigkeit kontaminieren. Primärbehälter sind zum z.B. Phiolen, Kartuschen, vorgefüllte Spritzen oder Ampullen. Primärbehälter können aus Glas, Kunststoff (z.B. Cycloolefin(co)polymer, Polyamid, Polypropylen) oder Metall (z.B. Edelstahl) bestehen. Primärbehälter sind geeignet für z.B. flüssige oder lyophilisierte pharmazeutische Produkte inklusive biologische pharmazeutische Produkte.

**"Pharmazeutische Produkte"** umfassen eine oder mehrere pharmazeutisch wirksame Agenzien wie z.B. chemische Moleküle, Peptide, Nucleinsäuren (z.B. mRNA oder DNA), Lipide, Proteine (z.B. Enzyme, Antikörper, Fusionsproteine), Viruspartikel oder Zellen. Pharmazeutische Produkte können z.B. flüssig oder lyophilisiert sein.

**"Biologische pharmazeutische Produkte"** umfassen eine oder mehrere biologische pharmazeutisch wirksame Agenzien wie z.B. Polynucleotide (z.B. mRNA oder DNA), Lipide, Proteine (z.B. Enzyme, Antikörper (z.B. Ranibizumab), Antikörper-Konjugate, Fusionsproteine (z.B. Aflibercept), Viruspartikel, Gewebeprodukte oder Zellen.

Der Begriff **"Indikatoren"** umfasst chemische und biologische Agenzien, welche auf einem Trägermaterial fixiert sind. Diese biologischen Agenzien können Viren, Pilze, Bakterien, Proteine (z.B. Enzyme), Nucleinsäuren, Kohlenhydrate oder Lipide sein. Als chemische Agenzien kommen meist Farbstoffe zum Einsatz, die per Farbumschlag den Sterilisationserfolg bestätigen. Als Trägermaterialen werden in der Regel Papier, Kunststoff, Edelstahl oder andere metallische Materialien verwendet. Einige Indikatoren, insbesondere für Trübungstests oder Prüfung auf Farbumschläge kommen aber in gelöster Form in durchsichtigen Behältern vor. Indikatoren sind in der Lage die Verminderung der Aktivität oder Menge einer Verunreinigung nach einer Sterilisation anzuzeigen.

Der Begriff **"biologische Indikatoren" oder "kommerzielle Indikatoren"** umfasst Indikatoren bei denen als biologisches Agens meist Bakterien bzw. deren Sporen (z.B. *Bacillus* oder *Geobacillus)* verwendet werden. Die Verminderung der wachstumsfähigen Menge an biologischem Agens nach der Sterilisation ist ein Maß für die Verminderung der Aktivität oder Menge einer Verunreinigung nach einer Sterilisation. Bioindikatoren können Gefäße mit einer Suspension sein oder aber auf einem festen Trägerstreifen fixiert sein.

Der Begriff **"Enzymindikatoren" oder "EI"** umfasst Indikatoren bei denen als biologisches Agenz Enzyme wie z.B. Kinasen (z.B. Adenylat-Kinase, Acetat-Kinase, Pyruvate-Kinase), verwendet werden. Das verwendete Enzym muss eine gegenüber dem Sterilisationsagens ausreichend hohe Stabilität aufweisen, um die fortschreitende Inaktivierung durch das Agens in einem Bereich anzuzeigen, der für die Prozessentwicklung nach dem Fraktion-Negativ-Verfahren oder Verfahren der Überlebenskurve erforderlich ist. Hierzu sollte die Inaktivierungskinetik der EIs der von biologischen Indikatoren als behördlich etablierten Referenzsystem möglichst ähnlich sein.

Die Verminderung der Enzymaktivität nach der Sterilisation ist ein Maß für die Verminderung der Aktivität oder Menge einer Verunreinigung nach einer Sterilisation. Prinzipiell sind daher alle Enzyme geeignet, die eine Reaktion katalysieren, deren Reaktionsprodukte in einem standardisierbaren Analysenverfahren nachgewiesen werden können. Solche Analyseverfahren können z.B. eine gekoppelten luminometrische Reaktionen sein, welche die Reaktionsprodukte umsetzt, photometrische Verfahren wie beispielsweise Fluoreszenzreaktionen oder die Messung eines Farbumschlags. So wird z.B. bei einer luminometrischen Reaktionen die Aktivität dieses Indikatorenzyms z.B. einer Kinase durch die Kopplung mit einer weiteren enzymatischen Reaktion einer Luciferase quantitativ ermittelt, wobei die Menge des durch die Luciferasereaktion emittierten Lichts proportional zur Aktivität des Indikatorenzyms ist. Reaktionsprodukte der enzymatischen Reaktion können aber auch mit anderen apparativen Verfahren wie beispielsweise chromatografisch bestimmt werden.

Die Enzyme könnten in ihrer nativen Form genutzt werden oder aber gentechnische modifiziert worden sein. Die Enzyme können einzeln oder in Kombination verwendet werden. Beispiele für geeignete Enzym-Indikatoren und deren Anwendung sind in WO2005/093085, WO2009/104013 oder WO2010/079357 beschrieben.

Als Trägermaterial kommt jedes Material in Betracht, dass eine ausreichend stabile Bindung zum jeweiligen Enzym ermöglicht und mit dem Sterilisationsagens nur im geringen Ausmaß wechselwirkt. Der Träger ist in Form, Abmessungen und Materialeigenschaften geeignet, um in das funktionale Packmittel eingebracht und darin fixiert werden zu können. Das Aufbringen der Indikatorenzyme auf ein Trägermaterial erfolgt in der Regel in Lösung. In Lösung gebrachtes Enzym wird in der Regel mittels einer Pipette oder automatisch dosiert aufgetropft.. Neben der rein adsorptiven Bindung sind auch klassische Einschlussverfahren wie der Enzymtechnologie denkbar.

### Verwendung der Enzymindikatoren

Ein erster Gegenstand der Erfindung ist eine Verwendung eines Enzymindikators für die Prozesskontrolle eines Sterilisationsverfahrens oder für die Entwicklung oder Validierung eines Sterilisationsverfahrens,
wobei das Sterilisationsverfahren geeignet ist, die Aktivität oder Menge einer Kontamination auf der Außenfläche eines Primärbehälters zu eliminieren und
wobei sich der Primärbehälter und der Enzymindikator in einem funktionalen Packmittel befinden.

Enzymindikatoren können dazu verwendet werden, einen Sterilisationsprozess zu entwickeln oder zu validieren. Dabei werden die EI dazu verwendet, den Sterilisationserfolg also die Verminderung der Menge oder Aktivität einer Kontamination in Abhängigkeit von verschiedenen Verfahrensparametern zu analysieren. Bei einem etablierten Sterilisationsverfahren können EI dazu verwendet werden, zu überprüfen, ob die vorgegebene Verminderung der Menge oder der Aktivität einer Kontamination nach der Sterilisation erreicht wurde. Für die Entwicklung, Validierung und Kontrolle wird jeweils der Enzymindikator zu einer kontaminierten Probe hinzugefügt und der Indikator und die Probe werden dem entsprechenden Sterilisationsprozess unterzogen. Nach Beendigung des Sterilisationsprozesses wird die Restenzymaktivität analysiert. Die Verringerung der Enzymaktivität durch die Sterilisation wird dann mit der Verringerung der Menge oder Aktivität der Kontamination korreliert. Wenn ein Aktivitätsniveau bestimmt wird, von dem bekannt ist, dass es mit einer akzeptablen Verringerung der Kontamination korreliert, gilt die Sterilisation als validiert.

Es ist möglich, dass auf der Außenfläche des Primarbehälter vor der Sterilisation keine Kontamination vorhanden ist. Der Sinn der Verwendung der Enzymindikatoren besteht darin, dass nach Durchführung des Sterilisationsverfahrens bestätigt wird, dass eine möglicherweise vorhandene Kontamination in einem akzeptablen Ausmaß entfernt/inaktiviert wurde. Die Reduktion der Enzymaktivität des Enzymindikators nach einem Sterilisationsverfahren korreliert mit der Verringerung der Menge oder Aktivität einer Kontamination auf der Außenfläche eines Primärbehälters. Im Allgemeinen wird jedoch davon ausgegangen, dass die Außenfläche kontaminiert ist, oder es wird vermutet, dass sie kontaminiert ist.

In einer Ausführungsform der erfindungsgemäßen Verwendung ist der Primärbehälters ein Medizinprodukt wie z.B. ein Implantat, chirurgische Instrumente, oder Verbandsmaterial.

In einer weiteren Ausführungsform der erfindungsgemäßen Verwendung entspricht die Enzymaktivität des Enzymindikators nach Anwendung des Sterilisationsverfahrens einer Reduktion einer Kontamination um mindestens 6 log oder mindestens 7 log oder mindestens 8 log oder mindestens 9 log oder mindestens 10 log oder mindestens 11 log oder mindestens 12 log. Besonders bevorzugt ist eine Verminderung der Enzymaktivität die einer Reduktion von mindestens 10 log oder mindestens 11 log oder mindestens 12 log entspricht.

In einer weiteren Ausführungsform der erfindungsgemäßen Verwendung umfasst das Sterilisationsverfahren die Verwendung von Sterilisationsagenzien, welche verdampft werden oder gasförmig sind.

In einer weiteren Ausführungsform der erfindungsgemäßen Verwendung besteht das funktionale Packmittel zumindest teilweise aus einem Material, das für die Verwendung eines Sterilisationsverfahrens geeignet ist, das Sterilisationsagenzien umfasst, welche verdampft werden oder gasförmig sind.

In einer weiteren Ausführungsform der erfindungsgemäßen Verwendung ist das funktionale Packmittel ein Blister ist.

In einer weiteren Ausführungsform der erfindungsgemäßen Verwendung besteht der Blister zumindest teilweise aus einem Material, welches Polyethylen oder Papier umfasst.

In einer weiteren Ausführungsform der erfindungsgemäßen Verwendung ist der Primärbehälter eine vorgefüllte Spritze.

In einer weiteren Ausführungsform der erfindungsgemäßen Verwendung umfasst der Primärbehälter ein pharmazeutisches Produkt.

In einer weiteren Ausführungsform der erfindungsgemäßen Verwendung umfasst der Primärbehälter ein biologisches pharmazeutisches Produkt.

In einer weiteren Ausführungsform der erfindungsgemäßen Verwendung umfasst das biologische pharmazeutische Produkt ein Protein, ein Polynucleotid, Zellen, Viruspartikel oder Gewebe.

In einer weiteren Ausführungsform der erfindungsgemäßen Verwendung ist das Protein ein Antikörper, Antikörper-Konjugat, Antikörperfragment, Enzym, oder ein Fusionsprotein.

In einer weiteren Ausführungsform der erfindungsgemäßen Verwendung ist der Enzymindikator eine Kinase, vorzugsweise eine Adenylat-Kinase.

### Sterilisationsverfahren unter Verwendung von Enzymindikatoren

Ein zweiter Gegenstand der Erfindung sind Verfahren zum Entwickeln oder Validieren eines Sterilisationsverfahrens oder zur Prozesskontrolle eines Sterilisationsverfahrens,
wobei das Sterilisationsverfahren geeignet ist, die Aktivität oder Menge einer Kontamination auf der Außenfläche des Primärbehälters zu eliminieren,
wobei sich der Primarbehälter in einem funktionalen Packmittel befindet, und wobei die Verfahren folgende Schritte umfassen:
   a) Einbringen eines Enzymindikators in das Innere des funktionalen Packmittels,
   b) Sterilisation des funktionalen Packmittels mit dem Sterilisationsverfahren,
   c) Entnahme des Enzymindikators aus dem funktionalen Packmittel nach Beendigung des Sterilisationsverfahrens,
   d) Bestimmen der Restaktivität des Enzyms und optional Berechnen der Verringerung der Enzymaktivität, und
   e) Vergleichen der Restaktivität des Enzyms mit einer Aktivität des Enzyms vor dem Sterilisationsverfahren oder Vergleichen der Restaktivität des Enzyms mit einem festgelegten Vergleichswert der Enzymaktivität.

Verfahren zum Entwickeln oder Validieren eines Sterilisationsverfahrens oder zur Prozesskontrolle eines Sterilisationsverfahrens verwenden Enzymindikatoren, um den Sterilisationserfolg also die Verminderung der Menge oder Aktivität einer Kontamination in Abhängigkeit von verschiedenen Verfahrensparametern zu analysieren. Bei einem etablierten Sterilisationsverfahren können EI dazu verwendet werden, zu überprüfen, ob die vorgegebene Verminderung der Menge oder der Aktivität einer Kontamination nach der Sterilisation erreicht wurde. Für die Entwicklung, Validierung und Kontrolle wird jeweils der Enzymindikator zu einer kontaminierten Probe hinzugefügt und der Indikator und die Probe werden dem entsprechenden Sterilisationsprozess unterzogen. Nach Beendigung des Sterilisationsprozesses wird die Restenzymaktivität analysiert. Die Verringerung der Enzymaktivität durch die Sterilisation wird dann mit der Verringerung der Menge oder Aktivität der Kontamination korreliert. Wenn ein Aktivitätsniveau bestimmt wird, von dem bekannt ist, dass es mit einer akzeptablen Verringerung der Kontamination korreliert, gilt die Sterilisation als validiert.

In einer weiteren Ausführungsform des erfindungsgemäßen Verfahrens entspricht die Enzymaktivität des Enzymindikators nach Anwendung des Sterilisationsverfahrens einer Reduktion einer Kontamination um mindestens 6 log oder mindestens 7 log oder mindestens 8 log oder mindestens 9 log oder mindestens 10 log oder mindestens 11 log oder mindestens 12 log. Besonders bevorzugt ist eine Verminderung der Enzymaktivität die einer Reduktion von mindestens 10 log oder mindestens 11 log oder mindestens 12 log entspricht.

*Schritt a):* Die Einbringung der Enzymindikatoren in das funktionale Packmittel erfolgt an einer Position und in einer Ausrichtung, welche die Außenfläche des Sterilisationsguts im funktionalen Packmittel angemessen repräsentiert. Das Verfahren zum Einbringen des EI ist geeignet, eine reproduzierbare Positionierung des EI zu ermöglichen. Geeignet ist z.B. die Nutzung von Schnittschablonen zur kontrollierten und reproduzierbaren Öffnung eines Teils des funktionalen Packmittels. Geeignet ist z.B. eine längliche Dimensionierung des Indikatorträgers, wobei das Enzym möglichst nah an einem der beiden Enden aufgebracht ist. Somit kann das Indikatorenzym durch den Schnitt im funktionalen Packmittel möglichst nah an die Oberfläche des Sterilisationsguts herangeführt werden. Zeitgleich steht das andere Ende des Trägers durch diese Dimensionierung derart aus dem funktionalen Packmittel heraus, dass der Träger mittels eines geeigneten Klebestreifens (Sterilisationsklebeband) fixiert und gleichzeitig der Schnitt im Packmittel versiegelt werden kann. Die Fläche des Klebebands sollte dabei so gewählt werden, dass einerseits eine sichere Fixierung und Schnittversiegelung erreicht wird, andererseits die Membraneigenschaften des funktionalen Packmittels gegenüber dem Sterilisationsagens möglichst wenig beeinträchtigt werden.

In einer weiteren Ausführungsform des erfindungsgemäßen Verfahrens umfasst das Sterilisationsverfahren die Verwendung von Sterilisationsagenzien, welche verdampft werden oder gasförmig sind.

In einer weiteren Ausführungsform des erfindungsgemäßen Verfahrens ist das Sterilisationsagenz Ethylenoxid, Formaldehyd, Stickstoffdioxid oder verdampftes Wasserstoffperoxid.

*Schritt c):* Erfolgt die Sterilisation mittels eines Sterilisationsagens, welches vom Material des funktionalen Packmittels adsorbiert wird wie z.B. Ethylenoxid oder Wasserstoffperoxid so wird in Schritt c), der Enzymindikator unmittelbar nach der Sterilisation aus dem funktionalen Packmittel entfernt, um eine weitere potentielle Inaktivierung durch desorbierendes Agens zu verhindern.

In einer weiteren Ausführungsform des erfindungsgemäßen Verfahrens erfolgt im Schritt c) die Entnahme des Enzymindikators unmittelbar nach Beendigung des Sterilisationsverfahrens, wenn das verwendete Sterilisationsagens vom Material des funktionalen Packmittels adsorbiert wird.

*Schritt d):* Die Analyse der Restaktivität des Enzyms nach Entnahme des Enzymindikators aus dem funktionalen Packmittel kann sofort oder später erfolgen. Wenn die Analyse der Restaktivität des Enzyms später erfolgt, wird der Träger mit dem Enzym gelagert. Die Lagerbedingungen hängen von der Art des Enzyms und seiner Fixierung auf dem Trägermaterial ab. So kann z.B. die Lagerung bei Raumtemperatur oder bei 4°C im Kühlschrank, vorzugsweise im Dunkeln erfolgen. Die Lagerung kann über mehrere Tage erfolgen, wie z.B. über bis zu 7 Tage.

In einer weiteren Ausführungsform des erfindungsgemäßen Verfahrens erfolgt im Schritt d) das Bestimmen der Restaktivität des Enzyms unmittelbar nach Schritt c) oder bis zu 7 Tagen nach Schritt c).

In einer weiteren Ausführungsform des erfindungsgemäßen Verfahrens erfolgt im Schritt d) das Bestimmen der Restaktivität des Enzyms bis zu 7 Tagen nach Schritt c)
wobei der Enzymindikator bei Raumtemperatur oder 4°C im Dunkeln bis zu 7 Tage nach Schritt c) gelagert wird.

*Schritt e):* Um den Sterilisationserfolg bewerten zu können, erfolgt ein Vergleich der Enzymaktivität vor dem Sterilisationsprozess mit der Restenzymaktivität nach dem Sterilisationsprozess. Die Verringerung der Enzymaktivität durch die Sterilisation oder die Restenzymaktivität wird dann mit der Verringerung der Menge oder Aktivität der Kontamination korreliert. Alternativ kann die Verminderung der Enzymaktivität mit der Aussage eines etablierten Bioindikators verglichen werden. Wenn ein Aktivitätsniveau bestimmt wird, von dem bekannt ist, dass es mit einer akzeptablen Verringerung der Kontamination korreliert, gilt die Sterilisation als validiert.

In einer weiteren Ausführungsform des erfindungsgemäßen Verfahrens ist der Enzymindikator eine Kinase, vorzugsweise eine Adenylat-Kinase.

### Funktionales Packmittel

Ein dritter Gegenstand der Erfindung sind funktionale Packmittel, welche mindestens einen Primärbehälter und mindestens einen Enzymindikator umfassen,
wobei das funktionale Packmittel die Sterilisation der Außenfläche des Primärbehälters mittels eines Sterilisationsverfahrens ermöglicht und
wobei das funktionale Packmittel die Sterilität des Primärbehälters mindestens über die Lebensdauer eines pharmazeutischen Produktes bewahrt.

Erfindungsgemäße funktionale Packmittel ermöglichen im Zusammenhang mit dem angewandten Sterilisationsverfahren und der erfindungsgemäßen Prozesskontrolle mit Enzymindikatoren folgende Punkte:
- eine Außenflächensterilisation eines Primärbehälters, insbesondere einer vorgefüllten Spritze, mit einer Verminderung von mindestens 6 log oder mindestens 7 log oder mindestens 8 log oder mindestens 9 log oder bevorzugt mindestens 10 log oder bevorzugt mindestens 11 log oder besonders mindestens 12 log
- schonende Sterilisationsbedingungen und somit die Sterilisation von Primärbehältern mit biologischen pharmazeutischen Produkten
- eine zeitnahe Prozesskontrolle und ein Nachweis des Sterilisationserfolges durch die Nutzung von Enzymindikatoren mit der beschriebenen Gebrauchsweise

Sterilisationsverfahren unter Verwendung von Sterilisationsagenzien, welche verdampft werden oder gasförmig sind, sind besonders geeignet für die Sterilisation von temperaturlabilen pharmazeutischen Produkten. Dabei ist es notwendig, dass das Sterilisationsagenz das funktionale Packmittel durchdringen kann, um die Außenfläche des Primärbehälters zu sterilisieren. Geeignete funktionale Packmittel sind die teilweise aus Materiealien wie z.B. Papier oder Polyethylen bestehen, die für das Sterilisationsagenz durchlässig sind. So kann z.B. ein Blister an einer Seite aus Papier bestehen.

In einer weiteren Ausführungsform des erfindungsgemäßen Packmittels besteht das funktionale Packmittels zumindest teilweise aus einem Material, das für die Verwendung eines Sterilisationsverfahrens geeignet ist, das Sterilisationsagenzien umfasst, welche verdampft werden oder gasförmig sind.

In einer weiteren Ausführungsform des erfindungsgemäßen Packmittels ist das funktionale Packmittel ein Blister.

In einer weiteren Ausführungsform des erfindungsgemäßen Packmittels besteht der Blister zumindest teilweise aus einem Material, welches Polyethylen oder Papier umfasst.

In einer weiteren Ausführungsform des erfindungsgemäßen Packmittels ist der Primärbehälter eine vorgefüllte Spritze.

In einer weiteren Ausführungsform des erfindungsgemäßen Packmittels umfasst der Primärbehälter ein pharmazeutisches Produkt.

In einer weiteren Ausführungsform des erfindungsgemäßen Packmittels umfasst der Primärbehälter ein biologisches pharmazeutisches Produkt.

In einer weiteren Ausführungsform des erfindungsgemäßen Packmittels umfasst das biologische pharmazeutische Produkt ein Protein, ein Polynucleotid, Zellen, Viruspartikel oder Gewebe.

In einer weiteren Ausführungsform des erfindungsgemäßen Packmittels ist das Protein ein Antikörper, Antikörper-Konjugat, Antikörperfragment, Enzym, oder ein Fusionsprotein ist.

In einer weiteren Ausführungsform des erfindungsgemäßen Packmittels ist der Enzymindikator eine Kinase, vorzugsweise eine Adenylat-Kinase.

### Abbildungen und Beispiele

Die Erfindung wird nun in spezifischen Ausführungsformen in den folgenden Beispielen und unter Bezugnahme auf die beigefügten Abbildungen beschrieben.

### Beschreibung der Abbildungen

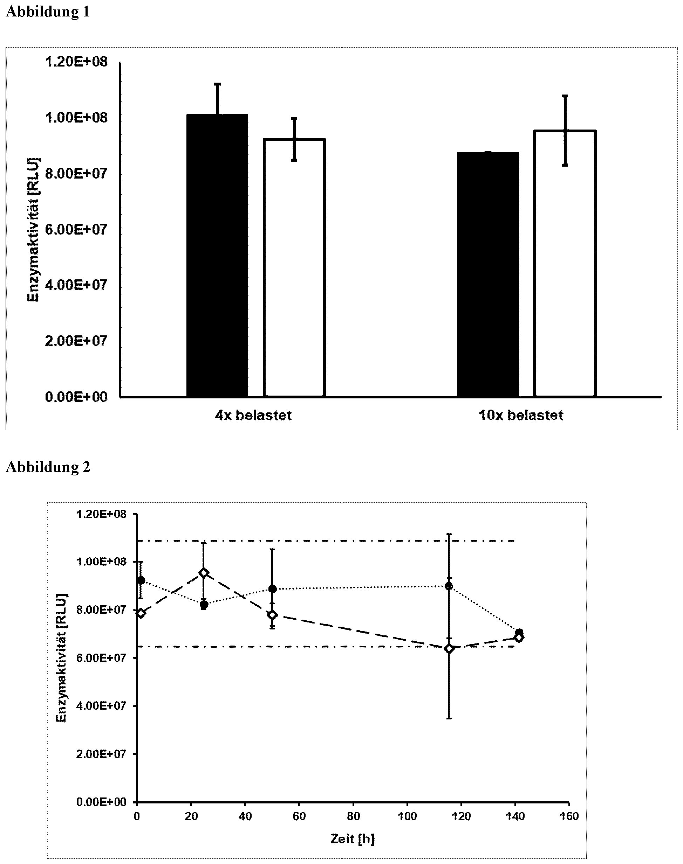

Abbildung 1: Enzymindikatoren wurden mechanisch, wie in Beispiel 1 beschrieben, belastet. Gezeigt ist die Enzymaktivität [RLU] nach 4-maliger Belastung (linke schwarze Säulen) bzw. 10-maliger Belastung (rechte schwarze Säule). Die Kontrollen wurden ohne vorausgehende Belastung dem luminometrischen Test unterzogen (weiße Säulen).

Abbildung 2: Enzymindikatoren wurden unterschiedlichen Lagerbedingungen ausgesetzt, wie in Beispiel 2 beschrieben. Gezeigt ist die Enzymaktivität [RLU] nach variabler Lagerdauer bei Raumtemperatur in Dunkelheit (schwarze Kreise) oder bei Exposition gegenüber Raumlicht (weiße Raute). Die horizontalen gestrichelten Linien geben den Bereich erwartbarer analytischer Variabilität des luminometrischen Tests an. Diese Variablilität basiert auf dem Bereich von drei Standardabweichungen um den Mittelwert der Positivkontrollen. Die hierzu genutzten Positivkontrollen wurden wie vom Hersteller empfohlen unter gekühlten und trockenen Bedingungen gelagert.

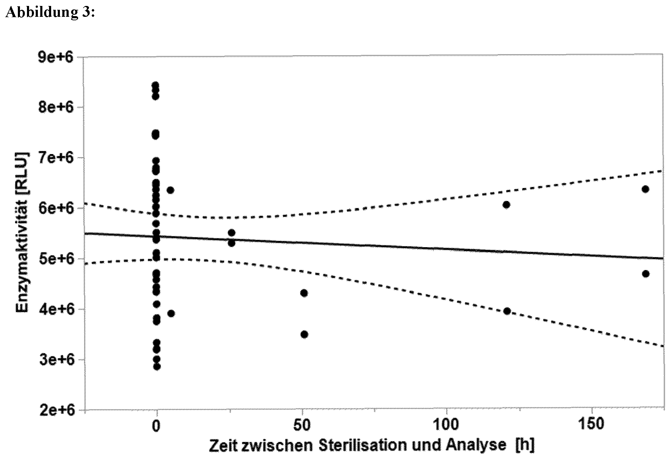

Abbildung 3: Enzymindikatoren wurden nach der Sterilisation unterschiedlich langer Lagerdauer bis zur Analyse im luminometrischen Testverfahren unterzogen, wie in Beispiel 3 dargestellt. Gezeigt ist die Enzymaktivität [RLU] nach variabler Lagerdauer nach Sterilisation. Die Lagerung begann nach Entnahme der Enzymindikatoren aus dem funktionalen Packmittel kurz nach der Sterilisation und erfolgte unter Lichtschutz und bei Raumtemperatur. Gezeigt ist weiterhin die Regressionslinie durch alle Datenpunkte (durchgehende Linie; p = 0,60) sowie das Konfidenzintervall der Regression (gestrichelte Linien; 95%).

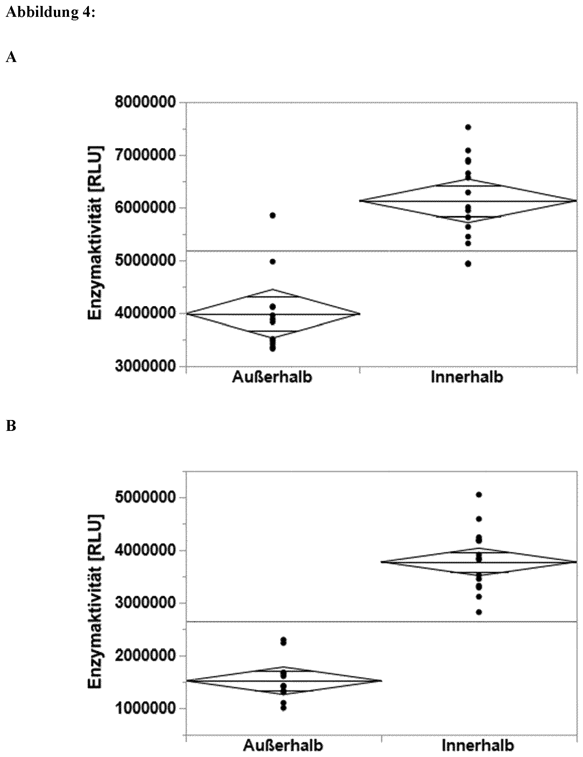

Abbildung 4: Enzymindikatoren wurden nach Sterilisation der luminometrischen Analyse unterzogen, wie in Beispiel 4 beschrieben. Gezeigt ist die Enzymaktivität [RLU] von Indikatoren, welche während der Sterilisation entweder innerhalb (linke Datenpunkte) oder außerhalb (rechte Datenpunkte) des funktionalen Packmittels positioniert waren. Die Abbildungen zeigen die Enzymaktivtäten nach vier (A) beziehungsweise elf (B) Injektionspulsen. In beiden Fällen ist die Enzymaktivitäten nach Sterilisation außerhalb des funktionalen Packmittels signifikant niedriger (p<0,0001) als bei Enzymindikatoren, welche sich während der Sterilisation innerhalb des funktionalen Packmittels befanden.

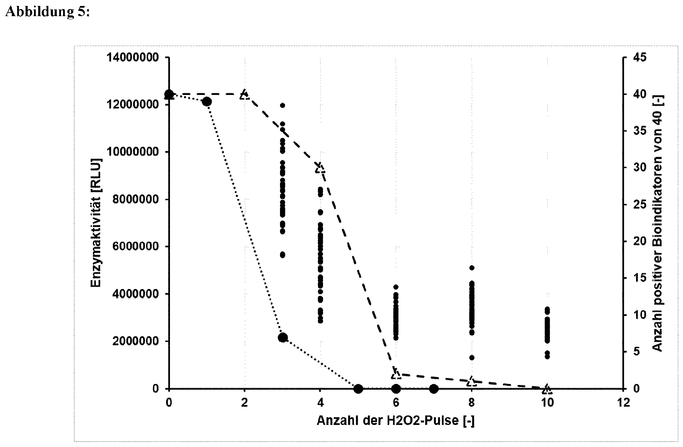

Abbildung 5: Die Inaktivierung von enzymatischen Indikatoren über eine steigende Anzahl von Injektionspulsen des Sterilisationsagens wurde der Inaktivierung biologischer Indikatoren zweier verschiedener Hersteller gegenübergestellt, wie in Beispiel 5 dargestellt. Gezeigt ist die Enzymaktivität [RLU] von Enzymindikatoren (linke y-Achse; einzelne Datenpunkte) über einer steigenden Anzahl and Injektionspulsen (x-Achse) sowie die Anzahl Wachstums-positiver Bioindikatoren von 40 platzierten Bioindikatoren zweier verschiedener Hersteller (rechte y-Achse; verbundene Dreiecke: Hersteller A, verbundene Kreise: Hersteller B).

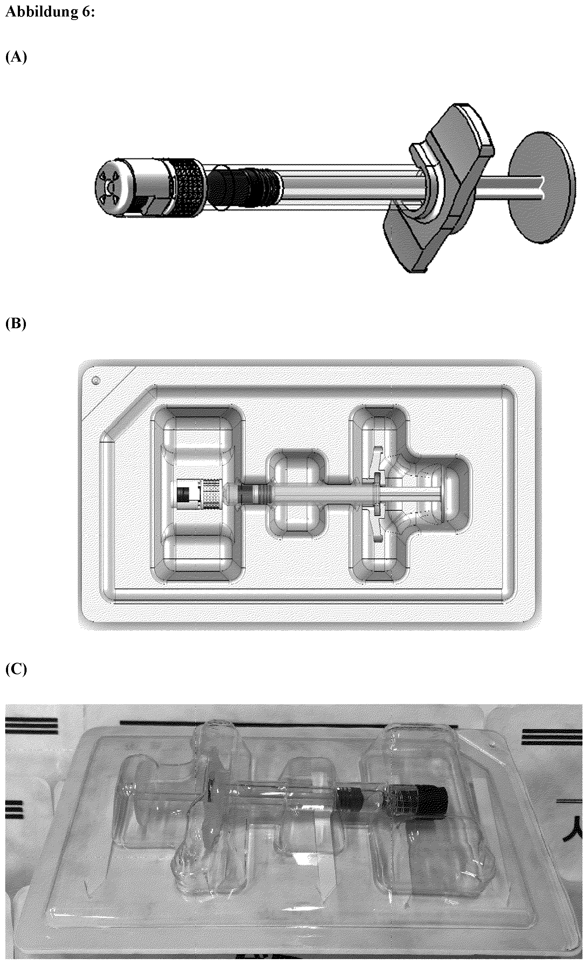

Abbildung 6: (A) zeigt eine vorgefüllte Spritze als Beispiel für einen Primärbehälter. (B) zeigt eine vorgefüllte Spritze in einem Blister als Beispiel für einen Primärbehälter in einem funktionalen Packmittel. (C) zeigt ein funktionales Packmittel mit einem eingebrachten EI, welcher mittig zwischen dem Kombinationsprodukt und der dampfdurchlässigen Membran des funktionalen Packmittels platziert ist.

### Tests zur Eignung von Enzymindikatoren

Die im folgenden beschriebenen Beispiele wurden mit kommerziell erwerblichen Enzymindikatoren der Firma Protak Scientific Ltd (Redhill, GB) verwendet. Hierbei ist auf einem Trägerstreifen aus Kunststoff eine thermostabile Adenylatkinase aufgebracht, deren graduelle Inaktivierung bei Kontakt mit verdampftem Wasserstoffperoxid über die Enzymaktivität in einem gekoppelten luminometrischen Testverfahren ermittelt wurde.

Unmittelbar nach Sterilisation wurden die Enzymindikatoren aus dem funktionalen Packmittel entfernt und dem von Protak Scientific Ltd erhältlichen luminometrischen Messverfahren zugeführt. Hierbei erfolgte die Messung der Enzymaktivität des auf dem Indikatorstreifen aufgebrachten Enzyms mittels eines gekoppelten, luminometrischen Enzymtests unter Verwendung von Luciferase. Der Enzymindikator wurde hierzu in ein transparentes Probenröhrchen gegeben, welches in die entsprechende Halterung des Luminometers eingesetzt wurde. Mit Beginn der Messung fuhr das Röhrchen in das Geräteinnere und sämtliche Reagenzien zur Bestimmung der Enzymaktivität mittels Luciferasereaktion wurden vollautomatisch zudosiert. Das in der Folge aufgezeichnete luminometrische Signal wurde in der entsprechenden Bediensoftware ausgegeben. Folgende Testreagenzien sowie folgendes Luminometer inklusive entsprechender Software von Protak Scientific Ltd. wurden eingesetzt:

| **Materialbezeichnung** | | **Herstellerbezeichnung** | | **Chargen-/Seriennummer** |
|---|---|---|---|---|
| Luminometer | | PR2A Twin Shot Reader | | 1064 |
| Luminometer Software | | Athena | | Version 1.1.0.0 |
| Reagenzien(-Kit), bestehend | | Protak kit: | | ATP Reagent SL: 250512 |
| aus: | | | - ATP Reagent SL | Diluent C: 250428 |
| | - Reaktionslösung | | - Diluent C | ADP Solution: 250603 |
| | - Verdünnungsmittel | | - ADP Solution | |
| | - ADP-Lösung | | | |
| Enzymindikator | | H₂O₂ tAK Enzyme Indicator Test | | 2004 |
| Probenröhrchen | | Luminescence Test Tube "LB 9517" | | 1126 |

Die gemessene Enzymaktivität wurde in relative Lichteinheit (RLU) angegeben. RLU ist ein relatives, nicht absolutes Maß, dass spezifisch für das verwendete optische Messsystem ist. Die Normung der RLU eines Messinstruments erfolgt im Rahmen der Gerätequalifizierung mittels einer kalibrierten Lichtquelle. Somit können Vergleiche zwischen verschiedenen Instrumenten angestellt werden.

### Beispiel 1: Beständigkeit gegenüber mechanischer Belastung

Indikatoren für funktionale Packmittel müssen in die funktionalen Packmittel eingebracht werden und können dabei mechanischen Belastungen ausgesetzt sein, wenn es zum Kontakt mit dem funktionalen Packmittel kommt. Es könnte somit Enzym abgetragen werden, was zu einer scheinbar gesenkten Enzymaktivität auf dem betroffenen Indikatorstreifen und somit einer Fehlinterpretation der Sterilisationskontrolle führen könnte. Es ist bekannt, dass BI durch solche mechanische Belastung falsch-positive Ergebnisse liefern können. Daher wurde getestet, ob EI dieser Belastung standhalten oder in ihrer Funktionalität beeinträchtigt werden.

Enzymindikatoren wurden hierzu mit der Hand 4 x bzw. 10 x über eine Papieroberfläche gezogen. Die Oberfläche, auf der das Enzym immobilisiert war, zeigte hierbei zur Papieroberfläche. Nach dieser Belastung wurden die Indikatoren dem oberhalb beschriebenen luminometrischen Testverfahren unterzogen und das Messergebnis wurde mit entsprechenden unbelasteten Kontrollen verglichen.

Selbst nach 10-maligem, druckvollem Ziehen des Enzymindikators über eine Papieroberfläche, zeigte sich kein relevanter Unterschied zur Kontrolle. Dies zeigt, dass die Enzymaktivität der EI nicht durch die übermäßige mechanische Belastung beeinträchtigt wird **(Fehler! Verweisquelle konnte nicht gefunden werden.).**

### Beispiel 2: Lagerstabilität der EI

Indikatoren müssen unter Umgebungsbedingungen ausreichend stabil sein, um während des Transportes und der Zwischenlagerung bis zum Einbringen in das Packmittel oder bis zur finalen Anwendung in der Sterilisation keinen relevanten Aktivitätsverlust aufzuweisen. Daher wurde die Lagerstabilität der EI getestet.

Hierzu wurden die oberhalb beschriebenen Enzymindikatoren bei Raumtemperatur sowohl unter Lichtschutz als auch unter Raumlicht gelagert und nach bis zu fünf Tagen Lagerung dem luminometrischen Aktivitätstest unterzogen.

Die getesteten EI zeigten über einen Zeitraum von 140 h lediglich einen marginalen Verlust der Enzymaktivität. Wurde dieser mit dem erwartbaren Bereich analytischer Schwankung verglichen (Mittelwert der Positivkontrollen der Messung +/- drei Standardabweichungen) so lagen alle gemessenen Aktivitäten der gelagerten Indikatoren im Bereich analytischer Variabilität. Dies zeigt, dass die Enzymaktivität der EI nicht durch die Lagerung bei Raumtemperatur bei Licht oder in Dunkelheit beeinträchtigt wurde (Abb. 2).

### Beispiel 3: Variabilität der Enzymaktivität nach der Sterilisation

Da es nicht immer möglich ist, die Enzymaktivität des EI sofort nach der Sterilisation zu messen, soll getestet werden, ob die Enzymaktivität über den Zeitraum der Lagerung nach der Sterilisation konstant bleibt.

Hierzu wurden insgesamt 50 Enzymindikatoren in je ein funktionales Packmittel eingebracht. Die derart präparierten Packmittel wurden im gesamten Raum der Sterilisationskammer verteilt platziert, um eine für das gesamte Sterilisationsgut repräsentative Probenmenge zu erzeugen. Die Sterilisation erfolgte durch Injektion von vier Pulsen verdampfter Wasserstoffperoxidlösung in eine teilevakuierte Sterilisationskammer. Jedem Puls folgte nach einer Haltezeit die erneute Teilevakuierung. Unmittelbar nach Sterilisation, wurden 40 Indikatoren dem Packmittel entnommen und die Enzymaktivität mittels des luminometrischen Testverfahren bestimmt. Die übrigen zehn Indikatoren wurden ebenfalls den Packmitteln entnommen und unter Ausschluss von Licht bei Raumtemperatur gelagert. Die weitere Bestimmung der Enzymaktivitäten erfolgte zu fünf verschiedenen Zeitpunkten jeweils im Duplikat. Die Ergebnisse der luminometrischen Messung wurden mittels Regressionsanalyse ausgewertet.

Der Einfluss der Lagerungszeit auf die Enzymaktivität ist analytisch nicht signifikant (p=0.60) Dies zeigte, dass die Enzymaktivität der EI nicht durch die Lagerung bei Raumtemperatur nach der Sterilisation beeinträchtigt wurde und das Ergebnis des luminometrischen Tests über bis zu sieben Tage ausreichend stabil war (Abb. 3). Somit ist sowohl eine zeitnahe Auswertung als auch eine zeitverzögerte Auswertung möglich. Die zeitverzögerte Auswertung von Indikatoren erlaubt die vereinfachte Prozesskontrolle im Zusammenhang mit Herstellprozessen im 24 Stunden/7 Tage-Betrieb, da die Anwesenheit von Personal zur Auswertung der EIs nicht 24 h /7 Tage gegeben sein muss, da die Auswertung ebenfalls am Folgetag erfolgen kann.

### Beispiel 4: Enzymaktivität nach Sterilisation innerhalb des funktionalen Packmittels

Es ist bekannt, dass EI den Erfolg einer Sterilisation gut anzeigen können, wenn die zu sterilisierende Oberfläche und der EI direkt dem Sterilisations-Agens ausgesetzt sind. Im Gegensatz dazu befinden sich bei der erfindungsgemäßen Verwendung der EI die zu sterilisierende Oberfläche und der EI in einem funktionalen Packmittel und sind somit dem Sterilisations-Agens nicht direkt ausgesetzt. Daher wurde der Einfluss des funktionalen Packmittels auf die Enzymaktivität analysiert.

Zur Gegenüberstellung der Enzymaktivität nach Sterilisation innerhalb und außerhalb des funktionalen Packmittels wurden somit jeweils 15 Enzymindikatoren entweder in ein Packmittel einbracht oder verblieben außerhalb. Jeweils fünf der Indikatoren innerhalb bzw. außerhalb des Packmittels wurden in enger räumlicher Nähe zueinander an drei verschiedenen Stellen innerhalb der Sterilisationskammer positioniert und, in Anlehnung an ein Fraktion-Negativ-Verfahren, einem verkürzten Sterilisationszyklus von entweder vier oder elf Injektionspulsen der Wasserstoffperoxidlösung unterzogen. Die Enzymaktivität aller Enzymindikatoren wurden unmittelbar nach der Sterilisation mittels des luminometrischen Testverfahrens bestimmt.

Es konnte gezeigt werden, dass die Enzymaktivität nach Sterilisation signifikant davon abhing, ob der EI innerhalb oder außerhalb eines Blisters positioniert war (Abb 4). Dies zeigte, dass das Einbringen der EI in den Blister zu einer deutlich unterschiedlichen Inaktivierungskinetik gegenüber dem bereits etablierten Verfahren der offenen Positionierung gegenüber des Sterilisationsgens führte. Die Auswirkung des funktionalen Packmittels als Diffusionsbarriere gegenüber dem Sterilisationsagens sowie Adsorptions- und Desorptionseffekte zwischen Packmittel und Sterilisationsagens müssen somit bei der Anwendung des hier dargestellten Verfahrens berücksichtigt werden. Erfolgt eine ausreichende Berücksichtigung wird jedoch die Anwendung enzymatischer Indikatoren in Kombination mit einem Oberflächensterilisationsverfahren eines Kombinationsproduktes in einem funktionalen Packmittel ermöglicht und der EI stellt die Erreichbarkeit der Oberfläche des Kombinationsprodukts gegenüber dem Sterilisationsagens identisch dar.

### Beispiel 5: Vergleich von EI mit BI

Es soll getestet werden, ob die Indikation des Sterilisationserfolgs mit EI vergleichbar zu BI ist, wenn sie sich in einem funktionalen Verpackungsmittel befinden.

Hierzu wurden entsprechend dem Fraktion-Negativ-Verfahren mehrere fraktionale Sterilisationszyklen mit unterschiedlicher Anzahl an Injektionspulsen (3, 4, 6, 8 oder 10 Pulse) durchgeführt. Hierzu folgte wie in Beispiel 3 beschrieben auf eine Teilevakuierung der Kammer die pulsweise Injektion von verdampftem Wasserstoffperoxid. In jedem Zyklus waren jeweils 40 Bioindikatoren und/oder 40 Enzymindikatoren einzeln in funktionalen Packmitteln (s. Abb. 6C) über die Sterilisationskammer verteilt. Um die bekannte Heterogenität zwischen Bioindikatoren unterschiedlicher Hersteller abzubilden, wurden Bioindikatoren zweier unterschiedlicher Hersteller (Indikatororganismus Geobacillus stearothermophilus; SKAN AG, Charge 190916-R, und Mesa Laboratories, Charge AS-025) verwendet.

Die Analyse der Enzymindikatoren erfolgte unmittelbar nach Entladung der Sterilisationskammer mittels des oberhalb beschriebenen luminometrischen Testverfahrens. Zur Auswertung der Bioindikatoren wurden diese innerhalb von vier Stunden nach Entladung der Sterilisationskammer unter Nutzung einer biologischen Sicherheitswerkbank in 10 mL eines geeigneten Nährmediums gegeben. Es wurde dabei darauf geachtete, dass der sporentragende Teil des Trägerstreifens nicht berührt wurde. Die Bebrütung erfolgte über sieben Tage bei 55-60°C. Zeigte sich nach spätestens sieben Tagen eine Eintrübung des Nährmediums, welche als Beleg für die Anwesenheit wachstumsfähiger Mikroorganismen gilt, so galt die Sterilisation für den jeweiligen Indikator als nicht bestanden. Als Positivkontrolle wurden Bioindikatoren mitgeführt, welche keinem Sterilisationsprozess ausgesetzt wurden.

Die Enzymindikatoren wiesen auch im verblisterten Zustand eine qualitativ und quantitativ ähnliche Inaktivierungskinetik wie biologische Indikatoren auf, mit dem Bereich fraktionaler Inaktivierung bei weniger als sechs Pulsen. Eine Validierung nach dem Fraktional-Negativ-Verfahren ist somit auch im vorliegenden Anwendungsfall möglich, da eine ähnliche Sensitivität des Indikatorsystems geben ist.

### Beispiel 6: Eine vorgefüllte Spritze als Beispiel für einen Primärbehälter

Eine typische vorgefüllte Spritze ist in Abb. 6A gezeigt.

Die vorgefüllte Spritze besteht aus einem zylindrischen Körper, welcher aus Glas oder Kunststoff (z.B. Cycloolefin oder Polypropylen). Der zylindrische Körper kann z.B. ein Innenvolumen von 0,3 ml bis 1 ml oder auch größer sein. Der zylindrische Teil kann am vorderen Ende als Luer Konus ausgeformt sein. Der zylindrische Teil kann am hinteren Ende mit einem Flansch versehen sein. Der zylindrische Teil kann mit einem Gleitmittel, wie z.B. Silicon, beschichtet sein. Der zylindrische Teil kann mit einer Skalierung oder einer Dosismarkierung bedruckt sein.

Die vorgefüllte Spritze ist am vorderen Ende (zum Medikamentenaustritt) mit einem Verschluss abgedichtet, der (zur Abdichtung aber nicht zwangsläufig komplett) aus einem Elastomer (wie Butyl- oder Polyisopren) oder einem thermoplastischen Elastomer besteht. Dieser Verschluss kann weitere Funktionen beinhalten, wie einem Sicherheitsverschluss oder einen Luer-Lock-Adapter.

Die vorgefüllte Spritze ist am hinteren Ende mit einem Verschluss abgedichtet, der (zur Abdichtung aber nicht zwangsläufig komplett) aus einem Elastomer (wie Butyl-, Polyisopren oder Polyisobutylen) oder einem thermoplastischen Elastomer besteht. Diese hintere Abdichtung kann ganz oder teils beschichtet sein, z.B. mit Polytetrafluorethylen oder Silikonöl, um die Eigenschaften zu optimieren. Die Abdichtung am hinteren Ende kann aus mehreren Materialien aufgebaut sein, wie z.B. einem Kunststoffkern mit Gewinde und einer weicheren Hülle.

In die Abdichtung am hinteren Ende kann ein Gestänge angebracht sein (z.B über ein Gewinde oder eine andere Passverbindung). Das Gestänge kann aber auch ohne feste Verbindung zur Abdichtung am hinteren Ende eingebracht sein. Mittels dieses Gestänges kann die Abdichtung bewegt und die Spritze über das vordere Ende entleert werden.

Am hinteren Ende kann ein zusätzlicher Flansch aufgesetzt werden, der eine bessere Handhabung ermöglicht. Dieser Flansch kann aus Kunststoff bestehen.

### Beispiel 7: Ein Blister umfassend eine vorgefüllte Spritze und einen Enzymindikator als Beispiel für ein funktionales Packmittel

Das Beispiel einer vorgefüllten Spritze im funktionalen Packmittel ist schematisch in Abbildung 6B dargestellt. Zur besseren Verdeutlichung ist hier auf die Darstellung der gaspermeablen Membran (z.B. aus Papier) verzichtet worden.

Der eigentliche Blister ist aus einem formbaren Kunststoff (z.B. Polyethylen) und ist derart ausgeformt, dass er dem eingebrachten Primärbehälter ausreichend Platz bietet, und zusätzlich über Kontaktstellen zum Spritzenkörper des Primärbehälters Halt gibt. Die Anzahl und Ausformung der Kontaktstellen ist auf das notwendige Maß für adäquaten Halt zu begrenzen, um keine Barriere für die Zugänglichkeit der Oberfläche gegenüber einem verdampften oder gasförmigen Sterilisationsagens darzustellen.

Abbildung 6C verdeutlich die mögliche Positionierung eines Indikatorstreifens innerhalb des funktionalen Packmittels. Die Einbringung des Indikators erfolgt durch lokales Einschneiden der gaspermeablen Membran. Der Indikatorstreifen wird dann vorsichtig eingeschoben, wobei das aus dem Blister herausragende Ende mit einem Klebestreifen fixiert wird, der zeitgleich den Schnitt in der Membran versiegelt. Der Ausrichtung des Indikators sollte hierbei immer gleich gehalten werden. In den hier gezeigten Beispielen zeigt die Enzym- oder Sporen-tragende Oberfläche genutzter Indikatoren immer in Richtung der gaspermeablen Membran.

## Patentansprüche

1. Verwendung eines Enzymindikators für die Prozesskontrolle eines Sterilisationsverfahrens oder für die Entwicklung oder Validierung eines Sterilisationsverfahrens,
wobei das Sterilisationsverfahren geeignet ist, die Aktivität oder Menge einer Kontamination auf der Außenfläche eines Primärbehälters zu eliminieren und
wobei sich der Primärbehälter und der Enzymindikator in einem funktionalen Packmittel befinden.

2. Die Verwendung des Enzymindikators
nach Anspruch 1
wobei die Enzymaktivität des Enzymindikators nach Anwendung des Sterilisationsverfahrens einer Reduktion einer Kontamination um mindestens 6 log oder mindestens 7 log oder mindestens 8 log oder mindestens 9 log oder mindestens 10 log oder mindestens 11 log oder mindestens 12 log entspricht.

3. Die Verwendung des Enzymindikators
nach Anspruch 1 oder 2
wobei die Enzymaktivität des Enzymindikators nach Anwendung des Sterilisationsverfahrens einer Reduktion einer Kontamination um mindestens 10 log oder mindestens 11 log oder mindestens 12 log entspricht.

4. Die Verwendung des Enzymindikators,
nach Anspruch 1, 2 oder 3
wobei das Sterilisationsverfahren die Verwendung eines Sterilisationsagenz umfasst, welches verdampft wird oder gasförmig ist.

5. Die Verwendung des Enzymindikators,
nach Anspruch 1, 2, 3 oder 4
wobei das funktionale Packmittels zumindest teilweise aus einem Material besteht, das für die Verwendung eines Sterilisationsverfahrens geeignet ist, das Sterilisationsagenzien umfasst, welche verdampft werden oder gasförmig sind.

6. Die Verwendung des Enzymindikators,
nach Anspruch 1, 2, 3, 4 oder 5
wobei das funktionale Packmittel ein Blister ist.

7. Die Verwendung des Enzymindikators,
nach Anspruch 1, 2, 3, 4, 5 oder 6
wobei der Blister zumindest teilweise aus einem Material besteht, welches Polyethylen oder Papier umfasst.

8. Die Verwendung des Enzymindikators,
nach Anspruch 1, 2, 3, 4, 5, 6 oder 7
wobei der Primärbehälter eine vorgefüllte Spritze ist.

9. Die Verwendung des Enzymindikators,
nach Anspruch 1, 2, 3, 4, 5, 6, 7 oder 8
wobei der Primärbehälter ein pharmazeutisches Produkt umfasst.

10. Die Verwendung des Enzymindikators,
nach Anspruch 1, 2, 3, 4, 5, 6, 7, 8 oder 9
wobei der Primärbehälter ein biologisches pharmazeutisches Produkt umfasst.

11. Die Verwendung des Enzymindikators,
nach Anspruch 1, 2, 3, 4, 5, 6, 7, 8, 9 oder 10
wobei das biologische pharmazeutische Produkt ein Protein, ein Polynucleotid, Zellen, Viruspartikel oder Gewebe umfasst.

12. Die Verwendung des Enzymindikators,
nach Anspruch 1, 2, 3, 4, 5, 6, 7, 8, 9, 10 oder 11
wobei das Protein ein Antikörper, Antikörper-Konjugat, Antikörperfragment, Enzym, oder ein Fusionsprotein ist.

13. Die Verwendung des Enzymindikators,
nach Anspruch 1, 2, 3, 4, 5, 6, 7, 8, 9, 10, 11 oder 12
wobei der Enzymindikator eine Kinase ist.

14. Verwendung des Enzymindikators,
nach Anspruch 1, 2, 3, 4, 5, 6, 7, 8, 9, 10, 11, 12 oder 13
wobei der Enzymindikator eine Adenylat-Kinase ist.

15. Ein Verfahren zum Entwickeln, Validieren eines Sterilisationsverfahrens oder zur Prozesskontrolle eines Sterilisationsverfahrens,
wobei das Sterilisationsverfahren geeignet ist, die Aktivität oder Menge einer Kontamination auf der Außenfläche des Primärbehälters zu eliminieren,
wobei sich der Primarbehälter in einem funktionalen Packmittel befindet, und
wobei das Verfahren folgende Schritte umfasst:
f) Einbringen eines Enzymindikators in das Innere des funktionalen Packmittels,
g) Sterilisation des funktionalen Packmittels mit dem Sterilisationsverfahren
h) Entnahme des Enzymindikators aus dem funktionalen Packmittel nach Beendigung des Sterilisationsverfahrens
i) Bestimmen der Restaktivität des Enzyms und optional Berechnen der Verringerung der Enzymaktivität und
j) Vergleichen der Restaktivität des Enzyms mit einer Aktivität des Enzyms vor dem Sterilisationsverfahren oder Vergleichen der Restaktivität des Enzyms mit einem festgelegten Vergleichswert der Enzymaktivität.

16. Das Verfahren zum Validieren eines Sterilisationsverfahrens oder zur Prozesskontrolle eines Sterilisationsverfahrens
nach Anspruch 15
wobei die Enzymaktivität des Enzymindikators nach Anwendung des Sterilisationsverfahrens einer Reduktion einer Kontamination um mindestens 6 log oder mindestens 7 log oder mindestens 8 log oder mindestens 9 log oder mindestens 10 log oder mindestens 11 log oder mindestens 12 log vermindert entspricht..

17. Das Verfahren zum Validieren eines Sterilisationsverfahrens oder zur Prozesskontrolle eines Sterilisationsverfahrens
nach Anspruch 15 oder 16
wobei die Enzymaktivität des Enzymindikators nach Anwendung des Sterilisationsverfahrens einer Reduktion einer Kontamination um mindestens 10 log oder mindestens 11 log oder mindestens 12 log entspricht..

18. Das Verfahren zum Validieren eines Sterilisationsverfahrens oder zur Prozesskontrolle eines Sterilisationsverfahrens,
nach Anspruch 15, 16 oder 17
wobei das Sterilisationsverfahren die Verwendung eines oder mehrerer Sterilisationsagenzien umfasst, welche verdampft werden oder gasförmig sind.

19. Das Verfahren zum Validieren eines Sterilisationsverfahrens oder zur Prozesskontrolle eines Sterilisationsverfahrens,
nach Anspruch 15, 16, 17 oder 18
wobei das Sterilisationsagenz Ethylenoxid, Formaldehyd, Stickstoffdioxid oder verdampftes Wasserstoffperoxid ist.

20. Das Verfahren zum Validieren eines Sterilisationsverfahrens oder zur Prozesskontrolle eines Sterilisationsverfahrens,
nach Anspruch 15, 16, 17, 18 oder 19
wobei im Schritt c) die Entnahme des Enzymindikators unmittelbar nach Beendigung des Sterilisationsverfahrens erfolgt, wenn das verwendete Sterilisationsagens vom Material des funktionalen Packmittels adsorbiert wird.

21. Das Verfahren zum Validieren eines Sterilisationsverfahrens oder zur Prozesskontrolle eines Sterilisationsverfahrens,
nach Anspruch 15, 16, 17, 18, 19 oder 20
wobei im Schritt d) das Bestimmen der Restaktivität des Enzyms unmittelbar nach Schritt c) oder bis zu 7 Tagen nach Schritt c) erfolgt.

22. Das Verfahren zum Validieren eines Sterilisationsverfahrens oder zur Prozesskontrolle eines Sterilisationsverfahrens,
nach Anspruch 15, 16, 17, 18, 19, 20 oder 21
wobei im Schritt d) das Bestimmen der Restaktivität des Enzyms bis zu 7 Tagen nach Schritt c) erfolgt und
wobei der Enzymindikator bei Raumtemperatur oder 4°C im Dunkeln bis zu 7 Tage nach Schritt c) gelagert wird.

23. Verfahren zum Validieren eines Sterilisationsverfahrens oder zur Prozesskontrolle eines Sterilisationsverfahrens,
nach Anspruch 15, 16, 17, 18, 19, 20, 21 oder 22
wobei der Enzymindikator eine Kinase ist.

24. Verfahren zum Validieren eines Sterilisationsverfahrens oder zur Prozesskontrolle eines Sterilisationsverfahrens,
nach Anspruch 15, 16, 17, 18, 19, 20, 21, 22 oder 23
wobei der Enzymindikator eine Adenylat-Kinase ist.

25. Ein funktionales Packmittel, welches mindestens einen Primärbehälter und mindestens einen Enzymindikator umfasst,
wobei das funktionale Packmittel die Sterilisation der Außenfläche des Primärbehälters mittels eines Sterilisationsverfahrens ermöglicht und
wobei das funktionale Packmittel die Sterilität des Primärbehälters mindestens über die Lebensdauer eines pharmazeutischen Produktes bewahrt.

26. Das funktionale Packmittel
nach Anspruch 25
wobei das funktionale Packmittels zumindest teilweise aus einem Material besteht, das für die Verwendung eines Sterilisationsverfahrens geeignet ist, das Sterilisationsagenzien umfasst, welche verdampft werden oder gasförmig sind.

27. Das funktionale Packmittel
nach Anspruch 25 oder 26
wobei das funktionale Packmittel ein Blister ist.

28. Das funktionale Packmittel
nach Anspruch 25, 26 oder 27
wobei der Blister zumindest teilweise aus einem Material besteht, welches Polyethylen oder Papier umfasst.

29. Das funktionale Packmittel
nach Anspruch 25, 26, 27 oder 28
wobei der Primärbehälter eine vorgefüllte Spritze ist.

30. Das funktionale Packmittel
nach Anspruch 25, 26, 27, 28 oder 29
wobei der Primärbehälter ein pharmazeutisches Produkt umfasst.

31. Das funktionale Packmittel
nach Anspruch 25, 26, 27, 28, 29 oder 30
wobei der Primärbehälter ein biologisches pharmazeutisches Produkt umfasst.

32. Das funktionale Packmittel
nach Anspruch 25, 26, 27, 28, 29, 30 oder 31
wobei das biologische pharmazeutische Produkt ein Protein, ein Polynucleotid, Zellen, Viruspartikel oder Gewebe umfasst.

33. Das funktionale Packmittel
nach Anspruch 25, 26, 27, 28, 29, 30, 31 oder 32
wobei das Protein ein Antikörper, Antikörper-Konjugat, Antikörperfragment, Enzym, oder ein Fusionsprotein ist.

34. Das funktionale Packmittel,
nach Anspruch 25, 26, 27, 28, 29, 30, 31, 32 oder 33
wobei der Enzymindikator eine Kinase ist.

35. Das funktionale Packmittel,
nach Anspruch 25, 26, 27, 28, 29, 30, 31, 32, 33 oder 34
wobei der Enzymindikator eine Adenylat-Kinase ist.
